(19) Europäisches Patentamt
European Patent Office
Office européen des brevets

(11) EP 1 945 182 B1

(12) **EUROPEAN PATENT SPECIFICATION**

(45) Date of publication and mention of the grant of the patent:
**28.07.2010 Bulletin 2010/30**

(21) Application number: **06850463.8**

(22) Date of filing: **13.10.2006**

(51) Int Cl.:
***A61K 9/08*** *(2006.01)* ***A61K 9/19*** *(2006.01)*
***A61K 31/4709*** *(2006.01)*

(86) International application number:
**PCT/IB2006/004228**

(87) International publication number:
**WO 2007/110709 (04.10.2007 Gazette 2007/40)**

(54) **FORMULATIONS OF TIPIFARNIB FOR INTRAVENOUS APPLICATION**

FORMULIERUNGEN VON TIPIFARNIB ZUR INTRAVENÖSEN VERABREICHUNG

Préparation de tipifarnib pour l'application intraveineuse

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.10.2005 US 726911 P**

(43) Date of publication of application:
**23.07.2008 Bulletin 2008/30**

(73) Proprietor: **Janssen Pharmaceutica N.V.**
**2340 Beerse (BE)**

(72) Inventors:
- **DE PORRE, Peter Marie-Zoe Robert**
**B-9000 Gent (BE)**
- **DRIES, Willy, Albert, Maria, Carlo**
**B-2330 Merksplas (BE)**
- **FRANCOIS, Marc, Karel, Jozef**
**B-2950 Kapellen (BE)**
- **PALMER, Peter, Albert**
**B-3140 Keerbergen (BE)**

(74) Representative: **Warner, James Alexander et al**
**Carpmaels & Ransford**
**43-45 Bloomsbury Square**
**London WC1A 2RA (GB)**

(56) References cited:
**WO-A-2005/052005** **WO-A-2006/081444**
**US-A1- 2005 136 063**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 945 182 B1

## Description

### Field of the invention

This invention concerns novel formulations of tipifarnib, which are suitable for intravenous (IV) administration. The invention further concerns the use of such formulation and processes for preparing such formulations and methods of treating patients by administering said formulations to said patients.

### Background of the invention

Tipifarnib is described in WO 97/21701. The chemical name for tipifarnib is (R)-(+)-6-[amino94-chlorophenyl)(1-mehtyl-1H-imidazol-5-yl)methyl]-4-(3-chlorophenyl)-1-methyl-2(1H)quinolinone. The trade name of tipifarnib is trade name ZARNESTRA® The absolute stereochemical configuration of the compound was not determined in the experiments described in the above-mentioned patent specification, but the compound was identified by the prefix "(B)" to indicate that it was the second compound isolated from column chromatography. The compound thus obtained has been found to have the (R)-(+)-configuration. This compound that is also referred to by its published code number R115777, has the following structure:

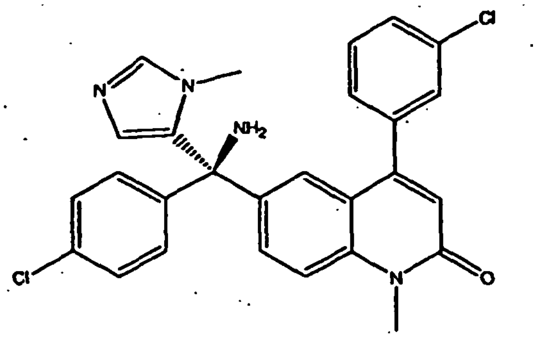

Tipifarnib is a base with very good water solubility at acidic pH. It hydrolyzes to R110127, wherein the amine functionality is replaced by a hydroxy functionality. The rate of the hydrolytic degradation is lowest at acidic condition but still very fast. R110127 has the following structure:

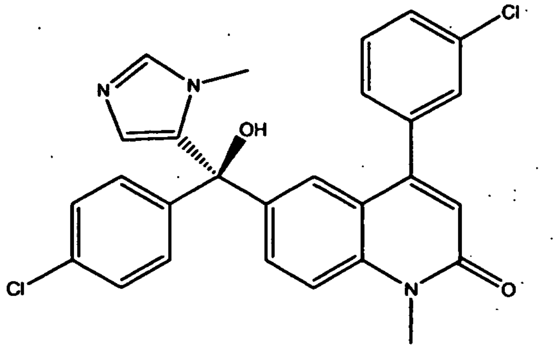

Tipifarnib is a potent and selective nonpeptidomimetic competitive inhibitor of human farnesyltransferase (FTase) in vitro and in vivo. This compound has antiproliferative effects at nanomolar concentrations in vitro, and has antitumor effects as monotherapy in several in vitro and in vivo models and in the clinic. The predominant antitumor effects of tipifarnib include inhibition of angiogenesis, induction of apoptosis, and direct antiproliferation.

Farnesylated targets, such as (but not limited to) Ras, RhoB, and phosphatidyl inositol-3 kinase ($PI_3K$)/serine-threonine kinase AKT that are involved in cellular homeostasis and proliferation are often mutated or dysregulated in AML. Inhibition of farnesylation prevents myeloid leukemic cell growth and progenitor colony formation in vitro. Leukemic cells obtained from cancer patients were significantly more sensitive to the growth-inhibitory effects of tipifarnib than normal bone marrow cells. The identification of the specific downstream effectors by which inhibition of farnesylation

results in antileukemic activity is a subject of ongoing research. Tipifarnib has shown signs of clinical activity in patients with hematologic malignancies including but not limited to acute myelogenous leukaemia, myelodysplastic syndrome and multiple myeloma and in patients with solid tumours including but not limited to glioblastoma and breast cancer.

To date, several investigators have examined the pharmacokinetics of tipifarnib after oral administration. A dose-escalation trial of tipifarnib was performed on 28 patients with advanced cancer. Within the 50- to 500-mg b.i.d. dose range, a peak concentration range of 68 - 1458 ng/mL was achieved between 2 to 5 hours after oral administration. A linear increase in the plasma concentrations of tipifarnib was observed. Trough and peak plasma concentrations of tipifarnib observed in this study were within the range of antileukemic activity.

Tipifarnib is extensively metabolized following oral administration. The data from in vitro studies with human hepatocytes indicated that tipifarnib undergoes direct glucoronidation. The experiments with diagnostic inhibitors and heterologous expression systems also revealed that CYP3A4 was a predominant metabolic pathway for tipifarnib compared to other CYP450 enzymes such as CYP2C19, CYP2A6, CYP2D6, and CYP2C8/9/10. The metabolites of tipifarnib were inactive as farnesyl transferase inhibitor and antiproliferative agent in several preclinical studies. However, the pharmacokinetics of its individual metabolites had not been studied in humans. Several metabolites of tipifarnib were found in plasma samples after [$^{14}$C]tipifamib was orally administered to healthy male subjects. These metabolites included a glucuronide conjugate of tipifarnib and metabolites formed via oxidative demethylation, deamination, and loss of the methyl-imidazole moiety. Glucuronidation of the parent compound is a major route of biotransformation. However, metabolism of tipifarnib following administration of this compound to cancer subjects has not been studied. In addition, potential differences in tipifarnib metabolite disposition that may occur after different routes of administration have not been systematically examined.

Moreover, cancer patients regularly suffer from impaired oral intake due to tumoral obstruction, a surgical procedure, concomitant oral infections, significant taste perversion or anorexia, or nausea and vomiting. Hence, there is still a desire for a formulation that allows for another route of administration, such as an intravenous administration.

## Summary of the invention

It is the objective of the present invention to provide an alternative route of administration (i.e., parenteral) of tipifarnib for patients for whom oral administration is problematic. The objective of the present invention was to provide intravenous route for the administration of tipifarnib, either as continuous infusion or as a shorter-duration infusion (shorter than one hour to several hours; once or more times per day) that can provide an adequate exposure to tipifarnib, allowing for similar clinical outcome as the current twice-daily oral administration of tipifarnib. In particular, the objective of the present invention was to provide intravenous route for the administration of tipifarnib, either as continuous infusion or as a twice-daily 2-hour infusion that can provide a similar exposure to the current twice-daily oral administration of tipifarnib.

## Brief description of the figures

FIGURE 1: "Table 1": Summary of the Demographic Data of the patients participating in the study.

FIGURE 2: "Table 2": Mean (± SD) Tipifarnib Plasma $C_{max}$ and $AUC_{0-10h}$ Values following Administration as 2-Hour Intravenous Infusion b.i.d.

FIGURE 3: "Table 3": Mean (± SD) Tipifarnib Plasma $C_{max}$ and $AUC_{0-10h}$ Values following Administration as Continuous Intravenous Infusion.

FIGURE 4: "Table 4": Systemic Exposure to Tipifarnib: 2 Hour Intravenous Infusion versus Oral Dosing.

FIGURE 5: "Table 5": Systemic Exposure to Tipifarnib: Continuous Intravenous Infusion versus Oral Dosing.

FIGURE 6: "Table 6": Mean (± SD) Plasma Pharmacokinetic Parameters of Tipifamib-Glucuronide Following Oral Administration, 2-Hour Intravenous Infusion, and Continuous Intravenous Infusion.

FIGURE 7: "Table 7": Mean (± SD) Plasma Pharmacokinetic Parameters of R130525 Following Oral Administration, 2-Hour Intravenous Infusion, and Continuous Intravenous Infusion.

FIGURE 8: Mean (± SD) Plasma Concentration-Time Profiles of Tipifarnib After consecutive Administration of Three 4-Day Treatment regimens.

FIGURE 9: Relationship between the Free Fraction and Total Plasma Concentrations of Tipifarnib.

FIGURE 10: Mean (± SD) Plasma Concentration-Time Profiles of Tipifarnib, and Derived Metabolites after Consecutive Administration of Three 4-Day Treatment Regimens.

**Description of the invention**

The present invention provides for an alternative route of administration (i.e., parenteral) of tipifarnib for patients for whom oral administration is problematic. The objective of the present invention was to provide intravenous route for the administration of tipifarnib, either as continuous infusion or as a shorter-duration infusion (shorter than one hour to several hours; once or more times per day) that can provide an adequate exposure to tipifarnib, allowing for similar clinical outcome as the current twice-daily oral administration of tipifarnib. In particular, the objective of the present invention was to provide intravenous route for the administration of tipifarnib, either as continuous infusion or as a twice-daily 2-hour infusion that can provide a similar exposure to the current twice-daily oral administrations of tipifarnib.

In this regard, the present invention provides a pharmaceutical formulation suitable for intravenous administration comprising tipifarnib, mannitol and hydroxypropyl-β-cyclodextrin.

A dry formulation of tipifarnib that can be solubilized with a solution so as to allow for a formulation of tipifarnib that can be administered intravenously is also described. Persons skilled in the art are aware of the limitations that are applicable to the formulations for intravenous administration. A number of limitations will be discussed hereinafter. The formulation that is administered intravenously should have a certain pH. The solution that is entered into the veins cannot be too basic nor can it be to acidic. The formulation should not contain excipients that would cause adverse reaction when entered into the blood. Furthermore, the formulation should allow for the active ingredient to remain soluble once entered into the blood. This list of limitations is not exhaustive.

The formulation of the present invention comprises tipifarnib in appropriate amounts. These amounts will depend upon whether the resulting IV formulation will be used for single dose administration or whether it will be used for continuous IV administration.

The formulation comprises further hydroxypropyl β-cyclodextrine and mannitol. Other cyclodextrines may also be used.

Optionally, another sugar, such as, for example, trealose or sucrose may be used. The mannitol ensures a quick solution of the lyophilized material. When the mannitol is not present some gel-like aggregates are observed that only slowly dissolve. With the mannitol present these aggregates do not appear or appear to a lesser extent. Moreover the mannitol ensures that the cosmetic aspect of the lyophilized material is improved. Without the mannitol the lyophilized material can show cracks that give the lyophilized material a less appealing appearance from a cosmetic point of view.

The pharmaceutical formulation of the invention may be administered to inhibit abnormal growth of cells, including transformed cells. Abnormal growth of cells refers to cell growth independent of normal regulatory mechanisms (e.g. loss of contact inhibition). This includes the abnormal growth of: (1) tumor cells (tumors) expressing an activated *ras* oncogene; (2) tumor cells in which the *ras* protein is activated as a result of oncogenic mutation of another gene; (3) benign and malignant cells of other proliferative diseases in which *ras* activation occurs. Furthermore, it has been suggested in literature that *ras* oncogenes not only contribute to the growth of tumors *in vivo* by a direct effect on tumor cell growth but also indirectly, *i.e.* by facilitating tumor-induced angiogenesis. Hence, pharmacologically targeting mutant *ras* oncogenes could conceivably suppress solid tumor growth *in vivo*, in part, by inhibiting tumor-induced angiogenesis.

This invention also provides formulations as defined in claim 1 for use the treatment of Acute myeloid leukemia.

This invention further provides formulations as defined in claim 1 for use in the treatment of breast cancer.

This invention further provides use of formulations as defined in claim 1 in the manufacture of a medicament for the treatment of Acute myeloid leukaemia.

This invention further provides use of formulations as defined in claim 1 in the manufacture of a medicament for the treatment of breast cancer.

<u>Terms & Definitions</u>

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of at least one of the symptoms of the disease or disorder being treated and / or reduction in the frequency and / or severity of at least one of the symptoms of the disease or disorder being treated.

The term "stereoisomer" refers to a isomers that have the same molecular formula and the same sequence of covalently bonded atoms but a different spatial orientation.

The term "optical isomer" means isomers of identical constitution that differ only in the spatial arrangement of their groups. Optical isomers rotate the plane of polarized light in different directions. The term "optical activity" means the degree to which an optical isomer rotates the plane of polarized light.

The term "racemate" or "racemic mixture" means an equimolar mixture of two enantiomeric species, wherein each of the isolated species rotates the plane of polarized light in the opposite direction such that the mixture is devoid of optical activity.

The term "enantiomer" means a stereoisomer that is not nonsuperimposable with its mirror image. The term "diastereomer" means stereoisomers that are not enantiomers.

The term "chiral molecule" means a molecule that has at least one pair of enantiomers. This is in contrast to achiral molecules which can be superimposed on their mirror images.

The two distinct mirror image versions of the chiral molecule are also known as levo (left-handed), abbreviated L, or dextro (right-handed), abbreviated D, depending on which way they rotate polarized light. The symbols "R" and "S" represent the configuration of groups around a stereogenic carbon atom(s).

An example of an enantiomerically enriched form isolated from a racemic mixture includes a dextrorotatory enantiomer, wherein the mixture is substantially free of the levorotatory isomer. In this context, substantially free means the levorotatory isomer may, in a range, comprise less than 25% of the mixture, less than 10 %, less than 5 %, less than 2 % or less than 1 % of the mixture according to the formula:

$$\%\,levorotatory = \frac{(mass\ levorotatory)}{(mass\ dextrorotatory) + (mass\ levorotatory)} \times 100$$

Similarly, an example of an enantiomerically enriched form isolated from a racemic mixture includes a levorotatory enantiomer, wherein the mixture is substantially free of the dextrorotatory isomer. In this context; substantially free means the dextrorotatory isomer may, in a range, comprise less than 25% of the mixture, less than 10 %, less than 5 %, less than 2 % or less than 1 % of the mixture according to then formula:

$$\%\,dextrorotatory = \frac{(mass\ dextrorotatory)}{(mass\ dextrorotatory) + (mass\ levorotatory)} \times 100$$

The term "geometric isomer" means isomers that differ in the orientation of substituent atoms in relationship to a carbon-carbon double bond, to a cycloalkyl ring, or to a bridged bicyclic system. Substituent atoms (other than hydrogen) on each side of a carbon-carbon double bond may be in an E or Z configuration. In the "E" configuration, the substituents are on opposite sides in relationship to the carbon-carbon double bond. In the "Z" configuration, the substituents are oriented on the same side in relationship to the carbon-carbon double bond.

Substituent atoms (other than hydrogen) attached to a ring system may be in a cis or trans configuration. In the "cis" configuration, the substituents are on the same side in relationship to the plane of the ring; in the "trans" configuration, the substituents are on opposite sides in relationship to the plane of the ring. Compounds having a mixture of "cis" and "trans" species are designated "cis/trans".

The isomeric descriptors ("R," "S," "E," and "Z") indicate atom configurations and are intended to be used as defined in the literature.

The compounds of the formulations of the invention may be prepared as individual isomers by either isomer-specific synthesis or resolved from an isomeric mixture. Conventional resolution techniques include combining the free base (or free acid) of each isomer of an isomeric pair using an optically active acid (or base) to form an optically active salt (followed by fractional crystallization and regeneration of the free base), forming an ester or amide of each of the isomers of an isomeric pair by reaction with an appropriate chiral auxiliary (followed by fractional crystallization or chromatographic separation and removal of the chiral auxiliary), or separating an isomeric mixture of either an intermediate or a final product using various well known chromatographic methods.

Furthermore, compounds of the formulations of the present invention may have one or more polymorph or amorphous crystalline forms and, as such, are intended to be included in the scope of the invention. In addition, some of the compounds may form solvates with water (i.e., hydrates) or common organic solvents (e.g., organic esters such as ethanolate and the like) and, as such, are also intended to be encompassed within the scope of this invention.

The following Examples are set forth to aid in the understanding of the invention, and are not intended and should not be construed to limit in any way the invention set forth in the claims which follow thereafter.

EXPERIMENTAL PART

**Formulations**

Tipifarnib 10 mg/ml injectable solution for lyophilization (Formulation 1)

| | | |
|---|---|---|
| tipifarnib | 10 | mg |
| mannitol | 50 | mg |
| concentrated hydrochloric acid | 3.75 | µl |
| hydroxypropyl-β-cyclodextrin | 200 | mg |
| water for injections qs ad | 1000 | µl |

Release pH = 2.0 ± 0.2

Solution for reconstituting tipifarnib injectable freeze dried powders (Formulation 2)

| | | |
|---|---|---|
| citric acid monohydrate | 1.97 | mg |
| disodium hydrogen phosphate anhydrous | 4.44 | mg |
| water for injections qs ad | 1000 | µl |

Release pH = 6.0 ± 0.5

**Preparation of the formulations.**

**Formulation 1**

10 ml of Formulation 1 is filled in a 20 ml vial and lyophilized (100 mg tipifarnib per vial). Before use the lyophilized powders are dissolved in 8.6 ml of Formulation 2 to obtain 10 ml of a tipifarnib 10 mg/ml injectable solution (pH ~ 4 - 5). This tipifarnib 10 mg/ml solution is then further diluted with a 0.9% NaCl solution to the desired concentration (0.5 to 2 mg/ml).

The pH value of Formulation 1 is very acidic (1) for solubilization of tipifarnib, (2) to have a minimal formation of R 110127 during manufacturing (manufacturing is done preferably at cold temperature ~ 10°C).

Hydroxypropyl-β-cyclodextrin is added to Formulation 1 to solubilize tipifarnib when it is reconstituted with Formulation 2. The pH value of the reconstituted solution is ~ 4.5. At this pH value tipifarnib precipitates if no Hydroxypropyl-β-cyclodextrin is present. The risk of local irritation during infusion into a peripheral vein (arm) is at an acceptable level at this pH value.

During the tests in humans some thrombophlebitis was found at the place of infusion when administered over longer periods of time (e.g. 24 hours). This may be caused by the hydroxypropyl-β-cyclodextrin itself and not by the vehicle. It was therefore decided not to administer via a peripheral vein but via a central vein. A central vein has a much higher blood debit so that mixing and dilution of the infusion solution is faster and higher. As the pH value of the infusion solution is less critical when infusion is done via a central vein, it was decided to reconstitute with WFI (water for injection) instead of with Formulation 2. The pH value of a solution obtained by reconstituting the lyophilized powders of Formulation 1 with WFI is ~3. The reconstituted solution can be administered via a central vein.

**Manufacturing process**

**(a) Manufacturing of Formulation 1 (10 L batch)**

(1) Add 2 kg of hydroxypropyl-β-cyclodextrin to a suitable container.
(2) Add 500 g mannitol to (1)
(3) Add 8 kg water for Injections to (2) and stir until solution
(4) Add 164.0 g HCl 10% to (3) and mix to homogeneous.
(5) Add 100 g R115777 to (4) and mix to solution.
(6) Filter through a sterile 0.22 μm filter.
(7) Fill 10 ml of (6) into 20 ml vials.

(b) Lyophilization of Formulation 1

(8) Stopper the vials and transfer to lyo trays.
(9) Transfer (8) to the lyophilizer.
(10) Lyophilize:

- freezing: - 40°C at atmospheric atmosphere
- primary drying: 10°C at 0.1 mbar
- secondary drying: 30°C at 0.01 mbar

(11) Crimping of the vials.

**Clinical trials**

**Methods and materials**

This randomized, open-label, comparative trial was performed in accordance with good clinical practice. Ethics Committee approval was obtained before the trial, and patients gave their written, informed consent to participate.

Tipifarnib was supplied as either 100-mg oral tablets or as lyophilized powders in vials containing100 mg tipifarnib and 2.0 g hydroxy-propyl-β-cyclodextrin (HPβCD). The lyophilized powders were reconstituted with Formulation 2 immediately prior to intravenous infusion. This aliquot was diluted in 0.9% NaCl and administered through a syringe driver with appropriate infusion lines.

**Study Subjects**

Men and women aged 18 years and older with pathologically confirmed cancer not amenable for curative therapy, were eligible for this study. Patients were required to have ECOG (Eastern Cooperative Oncology Group) Performance Statues ≤2. Other requirements included an absence of radiation therapy and/or chemotherapy at least 4 weeks prior to study entry (6 weeks for nitrosoureas or mitomycin C) and adequate oral intake to maintain a reasonable state of nutrition.

Patients were excluded if they had significantly abnormal hematological status as judged by a neutrophil count <1,500/$mm^3$, a platelet count <100,000/$mm^3$, serum bilirubin >2.0 mg/dL, transaminase >5 times the upper limit of institutional normal, or creatinine>1.5 mg/dL. Concomitant administration of proton pump inhibitors (e.g., omeprazole, lansoprazole, pantoprazole) was not allowed during the oral administration period of tipifarnib (this was only so in cycle 1, when PK assessment was done, to avoid any potential interference). Patients who underwent surgery of the gastrointestinal (GI) tract likely to interfere with drug absorption through impaired gastric acidity or deviation of the upper GI tract were not eligible. The presence of any concurrent disease that, in the opinion of the investigator, would constitute a hazard for participating in this study, and specifically grade ≥2 peripheral neuropathy also rendered a patient ineligible.

**Study Design**

The objective of the first (dose-determination) phase of the study was to identify an intravenous regimen that

was well tolerated. It was expected that this regimen would be equivalent to 50% - 60% of a clinically relevant oral dose (200 mg); based on a prior pilot determination of absolute bioavailability in healthy male volunteers. A first cohort of 3 subjects received the following uninterrupted 4-day consecutive treatments of tipifarnib: 30 mg as a 2-hour intravenous infusion b.i.d. on Day 1-4, followed by 200 mg as oral administration b.i.d. on Day 5-8, followed by 60 mg per day as a continuous intravenous infusion on Day 9-12. Similarly, a second cohort of 3 subjects received similar 4-day regimens of tipifarnib: 60 mg as a 2-hour intravenous infusion b.i.d., followed by 200 mg as oral administration b.i.d., followed by 120 mg per day as a continuous intravenous infusion. There was no washout period between each of the 4-day oral or i.v. treatments described.

The second and third subjects in the cohort were to be started only when the first subject completed the first intravenous and the oral treatment (i.e., 8-day period) without a dose-limiting toxicity (DLT). If a DLT occurred in 1 of 3 subjects of either the first or the second cohort, 3 supplementary subjects were to be added to that cohort. If more than 1 subject experienced DLT (either in the first 3 subjects or in the extended cohort of 6 subjects), dose escalation was to be terminated, and other subjects in that cohort had to complete their therapy at the same douse.

The second (i.v.-oral bridging) phase of the study was completed in separate group of patients. Systemic exposure to tipifarnib following the selected 2-hour and continuous intravenous regimens (from the previous dose-determination phase) was compared to exposure after a 200-mg b.i.d. oral regimen. The bridging phase was initiated when at least 2 subjects of the second cohort of the dose-determination phase were observed without DLT until Day 21. A total of 24 subjects were randomly assigned in a 1:1 ratio to one of two treatment sequences at the chosen intravenous regimens, in order to account for potential sequence-effects.

Sequence 1: 100 mg b.i.d. administered as a 2-hour intravenous infusion on Days 1-4, followed by 200 mg oral tablets b.i.d. on Days 5-8, then 200 mg/day administered as a continuous 4-day intravenous infusion on Days 9-12.

Sequence 2: 200 mg/day administered as a continuous 4-day intravenous infusion on Days 1-4, followed by 200 mg oral tablets b.i.d. on Days 5-8, then 100 mg b.i.d. administered as a 2-hour intravenous infusion on Days 9-12.

Oral doses of tipifarnib were to be given immediately following the intake of food.

The infusions of tipifarnib were administered either via a peripheral vein of the forearm or, for the higher doses, via a central line.

**Plasma Sampling and Assay**

In both the dose-determination and the bridging phases, pharmacokinetic sampling was performed during a 10-hour period from the start of the morning dose on Day 4 of each treatment. Whole blood samples were taken immediately before, and 0.5, 1, 2, 3, 4, 6, 8, and 10 hours after oral administration. For 2-hour intravenous infusion, blood samples were taken immediately before, and 1, 2 (i.e. just before the end of the infusion), 2.25, 3, 4, 6, 8, and 10 hours after dosing. Blood samples were taken immediately before, and 2, 4, 6, 8, and 10 hours after the start of the continuous intravenous infusion.

A minimum of 7 mL of blood for each PK sample was collected. Whole blood samples were immediately placed on ice and centrifuged within 2 hours after collection (10 min, 1000 x g). Separated plasma was frozen immediately at ≤ -18°C until assayed.

Plasma samples were thawed at room temperature, and individual sample was analyzed for tipifarnib using a previous published method. To 1-ml aliquots of human plasma, 200 ng of the internal standard (R121550, a structural analog) was spiked. After adding 1 ml of NaOH (0.1M), the samples were extracted twice with 4 ml of heptane containing 10 % of isoamylalcohol. The combined organic phases were back-extracted with 2 ml of HCl (2M). After alkalization with concentrated ammonia, the aqueous phase was extracted once with 5 ml of heptane containing 5% of isoamylalcohol. This extract was evaporated under nitrogen at 65°C and the residue dissolved in 100 microliters of injection solvent (Ammoniumacetate 0.01M/acetonitile/methanol; 50/25/25). A 15 μl extract was injected into an HPLC with UV detection (Agilent) at a wavelength of 240 nm. Separation was performed on a 10 cm x 4.6 mm chromatographic column, packed with 3 μm C18 BDS-Hypersil (Alltech). Elution was initially isocratic at 0.01M ammonium acetate / acetonitrile (52/48) until elution of the compounds of interest, followed by a gradient to 90% acetonitrile at a flow rate of 0.8 ml/min. The total run time was 14 min. The assay was validated in the range 2.00 to 5000 ng/ml.

The metabolites of tipifarnib, in particular R 130525, R 107252, and R 104209 were determined using LC-MS/MS. The chemical structures of these metabolites are represented in FIGURE 11. To determine the presence of glucuronidated metabolites, the plasma samples were also analyzed after deconjugation with *Escherichia Coli* β-*glucuronidase* (Boehringer Ingelheim GmbH, Ingelheim, Germany). The sample preparation was as in the following description: Aliquots of 0.2 mL human plasma or 1.0 mL aliquots of the diluted, deconjugated plasma samples were spiked with a mixture of 3 internal standards at 5 ng each: a stable isotope-labeled compound, R198838, used for tipifarnib and R130525 and two structural analogs, R107252 and R121704, used for R101763 and R104209, respectively. After adding 1 mL of 0.1M NaOH, samples were extracted twice with 3 mL of heptane, containing 10% isoamyl alcohol. The combined organic fractions were evaporated under nitrogen and the residue was reconstituted in a mixture of 100 μL, of methanol

and 100 μL of 0.002 M ammonium acetate. Chromatographic separation was done on a $C_{18}$ BDS-Hypersil 3 μm (3.2 mm I.D. x 50 mm) column. The injection volume was 25 μL. The isocratic mobile phase was composed of 0.002 M ammonium formate (pH 4 with formic acid)/acetonitrile (44/56) and pumped at 0.5 mL/minute. Detection was by tandem mass spectrometry with Turboionspray ionization (positive ion mode). The following mass transitions were used for monitoring the different compounds and internal standards: tipifarnib: 489.1 → 407.1; R 198838: 492.1 → 407.1; R 130525: 475.1 → 393.1; R101763: 394.0 → 139.0; R104209 + R107252: 408.0 → 139.0 and R121704: 422.1 → 139.0. The assay was validated in the range 0.50-1250 ng/ml for each of the metabolites and 10:0-25000 ng/ml for tipifarnib after deconjugation.

### Tipifarnib Protein Binding Analysis

Tipifarnib was specifically labeled with [$^{14}$C] at the asymmetric carbon atom. A 7-mL blood sample was taken from 24 patients at 4 hours after the start of the continuous intravenous infusion of tipifarnib on Day 4. The plasma samples were fortified with [$^{14}$C]-tipifarnib at 500 ng/ml. The fortified plasma samples were subjected to equilibrium dialysis against a 0.067 M phosphate buffer, pH 7.17 at 37°C for 4 hours in a Dianorm system with identical macro-1 Teflon cells and Diachema 10.17 dialysis membranes (MW cut-off 10,000). Radioactivity levels were measured in duplicate 100-μl aliquots of the fortified plasma before and after equilibrium dialysis, and in 1000-μl duplicate aliquots of the contents of buffer compartments of the dialysis cells admixed with methanol after dialysis in a Packard Tri-Carb 1900 TR liquid scintillation spectrometer. The total protein concentration was determined with a colorimetric biureet method, kit Roche Diagnostics 1929917. The albumin concentration was determined with a colorimetric bromocresol green method kit Roche Diagnostics 1970909. The $\alpha_1$-acid glycoprotein concentration was determined with an immuno-turbidimetric method, kit Roche Diagnostics 1557602. All these tests were performed on a Roche Hitachi Modular analyser.

The fraction of unbound tipifarnib ($f_u$), was calculated as the ratio of the unbound concentration ($C_u$) to the total concentration (C) as determined by radioactivity measurements in the buffer ($C_u$) and plasma (C) compartments of the dialysis cells: $f_u = C_u/C$. The bound fraction $f_b = 1 - f_u$.

### Noncompartmental Pharmacokinetic Analysis

Individual plasma concentration-time data of tipifarnib during the 10-hour period from the start of the morning dose on Day 4 of each administration period were analyzed by non-compartmental methods using the program WinNonlin version 3.1 (Pharsight Corporation, Cary, NC). The area under the concentration-versus-time curve ($AUC_{0-10h}$) from time 0 to 10 hours was calculated using the linear trapezoidal method. Maximum plasma concentrations ($C_{max}$), plasma concentration at steady state ($C_{ss}$, only for the continuous intravenous infusion), and time to reach the peak plasma concentration ($t_{max}$) were the observed values. The bioavailability of tipifarnib after oral administration was calculated based on $AUC_{0-10h}$ values according to $[AUC_{po} \times DOSE_{iv}/(AUC_{iv} \times DOSB_{po})] \times 100\%$.

### Statistical Analysis

Demographic information was summarized as median, minimum, and maximum. The pharmacokinetic data of tipifarnib and its metabolites were also summarized using descriptive statistics. The systemic exposures (plasma $C_{max}$ and $AUC_{0-10h}$) to tipifarnib after oral and intravenous administrations from the subjects whose pharmacokinetic samples for all 3 treatments were available were evaluated using ANOVA. The point estimates and corresponding 90% confidence intervals around the least squares mean ratio of intravenous infusion (test) versus oral administration (reference) for tipifarnib $C_{max}$ and $AUC_{0-10h}$ were calculated. The analysis was performed on logarithmically-transformed pharmacokinetic parameters using the SAS version 6.12 statistical software program (SAS Institute, Cary, NC).

### Clinical Assessment

Complete patient history was taken, and a physical examination (including vital signs), hematological tests, and clinical chemistry tests were performed at baseline and during treatment. To assess cardiovascular safety, twelve-lead E.C.G.s were recorded at screening, during Cycle 1 and at the end of therapy. Although objective tumor response was not the primary objective of this study, an attempt was made to obtain appropriate measurements to assess response. All adverse events and clinically relevant laboratory abnormalities were graded according to Common Toxicity Criteria (CTC version 2.0)

## RESULTS

### Subject Disposition

A total of 32 subjects (10 male and 22 female), were enrolled; 6 subjects participated in the dose-determination phase and 26 subjects in the bridging phase. Thirty-one subjects in this study were diagnosed with advanced solid tumor. One subject had high-risk myelodysplastic syndrome. In the bridging phase, 12 subjects were randomly assigned to receive Treatment Sequence 1 (2-hour intravenous infusion b.i.d./oral administration b.i.d./continuous intravenous infusion) and 14 subjects received Treatment Sequence 2 (continuous intravenous infusion/oral administration b.i.d./2-hour intravenous infusion b.i.d.). One subject randomized to Treatment Sequence 1 was withdrawn from the study on Day 4 because of a protocol violation and one subject on Treatment Sequence 2 was withdrawn on Day 8 due to hepatic failure related to disease-progression.

Demographic data of the 32 subjects recruited to the study are summarized in Table I (FIGURE 1). The age of patients ranged from 35 to 70 years, the weight range was 48 to 92 kg, and the height range was 149 to 176 cm. There were no obvious differences between the 2 randomized groups of Cohort 3 (bridging phase).

### Determination of the Intravenous Dose of Tipifarnib

The first cohort consisted of 3 subjects who received 4-day tipifarnib regimens of 30 mg twice daily as a 2-hour intravenous infusion followed by 200 mg twice daily as an oral administration and 60 mg per day as a continuous infusion. On average, systemic exposures to tipifarnib based on $AUC_{0\text{-}10h}$ for 30 mg b.i.d. as a 2-hour intravenous infusion and 60 mg per day as a continuous intravenous infusion were approximately 64% and 59% lower, respectively, relative to the exposure following a 200 mg oral dose received by these individuals (Tables II to IV) (FIGURES 2 to 4). No dose limiting toxicity after intravenous administration was observed. Therefore, the intravenous administration dose was escalated to the next dose level.

In the second cohort, 3 additional subjects received 4-day regimens of 60 mg tipifarnib twice daily as a 2-hour intravenous infusion followed by 120 mg per day as continuous infusion. Systemic exposures to tipifarnib following these intravenous regimens were approximately 47% and 46% lower, respectively, relative to the exposure following, a 200 mg oral dose (Tables II and IV) (FIGURES 2 to 4). Again, no DLT was observed in this cohort. Based on these results, a twice-daily regimen of 100 mg administered as a 2-hour infusion and a 200-mg/day continuous infusion were chosen for the intravenous to oral bridging study.

### Plasma Pharmacokinetics of Tipifarnib

As expected, the shape of the tipifarnib plasma concentration-time profile was dependent on the route of administration and the dosing regimen (Figure 1). Peak plasma concentrations of tipifarnib with apparent after oral administration and the 2-hour intravenous infusion. In contrast, continuous intravenous infusion produced relatively constant plasma concentrations during drug administration with no obvious peak.

Maximum plasma concentrations were observed at approximately 2 hours after oral administration of 200 mg b.i.d. of tipifarnib for 4 consecutive days (Table IV) (FIGURE 4). The range of $t_{max}$ was 0.5 to 3 hours in 29 subjects; 1 subject had a maximum concentration at 8 hours. The arithmetic mean $C_{max}$ and $AUC_{0\text{-}10h}$ values ($\pm$SD, n=24), after 4 days of b.i.d. oral dosing were 994 $\pm$ 487 ng/mL and 3990 $\pm$ 1671 ng·h/mL, respectively.

Higher plasma concentrations of tipifarnib were observed when a 100 mg dose was given as a 2-hour intravenous infusion relative to a 200 mg oral dose (Table IV) (FIGURE 4). On average, the $C_{max}$ and $AUC_{0\text{-}10h}$ values were 48.0% and 19.3% higher, respectively, when tipifarnib was administered as a 2-hour intravenous infusion (100 mg b.i.d.) than when administered orally (200 mg b.i.d.) (Table IV) (FIGURE 4). The upper limit of the 90% confidence interval fell outside the 80% to the 125% range of bioequivalence. The higher exposure observed following the 2-hour intravenous infusion compared with that following oral administration is not expected to be clinically significant.

The mean $AUC_{0\text{-}10h}$ values on Day 4 were similar both after oral administration (200 mg b.i.d.) and continuous intravenous infusion (200 mg per day, Table V) (FIGURE 5). The lower limit of the 90% confidence interval fell within the 80% to 125% range; however, the upper limit extended slightly beyond this range (Table V) (FIGURE 5). In terms of total systemic exposure as measured by the $AUC_{0\text{-}10h}$, the effect of dosing by continuous infusion was similar to that of dosing by the oral route. However, the shape of the profiles was different in that no peak tipifarnib concentration was apparent following the continuous infusion.

After dose-normalization, the mean absolute bioavailability of the oral tablet formulation (oral versus 2-hour i.v.) was 46.3%. Similarly, the mean absolute bioavailability (oral versus continuous intravenous infusion) of the oral tablet formulation was 40.9 %.

Systemic exposure to tipifarnib was comparable following administration as continuous intravenous infusion

(200 mg/day) or as 2-hour intravenous infusion (100 mg b.i.d.). Again, despite the differences in the shape of the plasma concentration-time profiles, the mean $AUC_{0-10h}$ values were similar. The upper and lower limits of the 90% confidence interval (81.1% to 100.3%) fell within the 80% to 125% range.

In summary, systemic exposure to tipifarnib as measured by plasma $AUC_{0-10h}$ during the 10-hour interval following dosing was comparable when this compound was administered as oral tablets (200 mg b.i.d.), by 2-hour intravenous infusion (100 mg b.i.d.), or by continuous intravenous infusion (200 mg/day). A narrow range of mean $AUC_{0-10h}$ values was observed across the 3 treatments (3990 to 4487 ng·h/mL). The 2-hour intravenous infusion more closely resembled oral administration when profile shape was considered.

**Protein Binding of Tipifarnib in Plasma**

Tipifarnib was extensively bound to plasma proteins in all samples analyzed (Figure 2). The mean free (unbound) fraction was 0.62% (range 0.45 to 0.88%) in the plasma samples drawn 4 hours after the start of the continuous intravenous infusion. The fraction of unbound tipifarnib was independent of total drug plasma concentration in the range of 211 ng/mL to 812 ng/mL. No data are available on protein binding from subject samples taken after oral administration or after 2-hour intravenous infusion. However, the median plasma concentration after 200 mg b.i.d. oral administration for 4 days during the 10-hour dose interval is in the range of 159 ng/mL to 788 ng/mL.

The mean concentrations (range) of serum albumin, ($\alpha_1$-acid glycoprotein, and total protein were 4.1 g/100 mL (3.1 to 4.7 g/mL), 131 mg/100 mL (90 to 218 mg/100 mL), and 6.8 g/100 mL (4.7 to 7.9 g/mL), respectively.

**Metabolites of Tipifarnib in Plasma**

Plasma pharmacokinetics of tipifarnib metabolites were analyzed on Days 4, 8, and 12 following 4 days administration as oral tablet (200 mg b.i.d.), 2-hour intravenous infusion (60 and 100 mg b.i.d.), or continuous intravenous infusion (120 and 200 mg/day). The metabolites analyzed included R130525, R101763, R104209, tipifarnib-glucuronide, R130525-glucuronide, and R104209-glucuronide. The chemical structures of these metabolites were demonstrated by Garner RC *et al*. The major metabolites observed in the systemic circulation were R130525 and the tipifarnib-glucuronide (Figure 3). The major and minor metabolites of tipifarnib determined in intravenous routes were the same as those in oral administration.

In general, systemic exposure ($C_{max}$ and $AUC_{0-10h}$) to the tipifarnib-glucuronide metabolite increased with an increase in the tipifarnib dose administered intravenously (Table VI) (FIGURE 6). Comparing in parallel, the exposure to the tipifarnib-glucuronide metabolite on Day 12 did not accumulate from previous 11-day administration (data are not shown). The mean ratio of $AUC_{0-10h}$ values for tipifarnib-glucuronide relative to tipifarnib was 18.6 for oral administration, 7.5 for 100 mg 2-hour intravenous infusion, and 8.6 for the 200 mg continuous intravenous infusion. Overall, the concentration ratio of glucuronide to the parent compound in plasma was higher after oral dosing of tipifarnib relative to intravenous administration. This is probably attributable to the first-pass effect through the liver (i.e., loss of the parent compound prior to systemic absorption) associated with dosing via the oral route.

Systemic exposure ($C_{max}$ and $AUC_{0-10h}$) to the R130525 metabolite increased with an increase in the tipifarnib dose intravenously administered (Table VII) (FIGURE 7). The mean ratio of $AUC_{0-10h}$ values for the R130525 relative to tipifarnib was one-fifth for the oral administration and approximately one-tenth for 2-hour intravenous infusion and continuous intravenous infusion.

Other metabolites of tipifarnib following oral administration of 200 mg tipifarnib were also measured in plasma including R101763 ($C_{max}$: 14.8 ng/mL, $AUC_{0-10h}$: 101 ng·h/mL), R104209 ($C_{max}$: 69.7 ng/mL, $AUC_{0-10h}$: 447 ng·h/mL), R130525-glucuronide ($C_{max}$: 53.0 ng/mL, $AUC_{0-10h}$: 320 ng·h/mL), R10176-glucuronide ($C_{max}$: 12.0 ng/mL, $AUC_{0-10h}$: 63.9 ng·h/mL:), and R104209-glucuronide ($C_{max}$: 64.0 ng/mL, $AUC_{0-10h}$: 434 ng·h/mL). The concentrations of these metabolites were much lower than those of R130525 and tipifarnib-glucuronide. The exposure to these metabolites following the intravenous administrations was lower than those following the oral administration.

**Clinical Assessment**

All subjects were evaluable for toxicity. One subject was withdrawn after 3 days as it was discovered she was not eligible for the study; no adverse events were reported. One subject had myelodysplastic syndrome. This subject had periods of grade 3-4 neutropenia, managed by dose reductions and delays. Platelet counts remained normal. No significant non-hematological toxicity was observed.

Grade 3 or 4 drug-related hematological toxicity, neutropenia, was observed in 9/30 patients (30%0. Febrile neutropenia was reported in 1 subject (3%). The main drug-related non-hematological toxicities, mainly grade1, were fatigue (60%), nausea (40 %), anorexia (20 %), vomiting (17 %) and diarrhea (13 %). Phlebitis was observed in 3 subjects, all when IV bolus was given via a peripheral venous access; this was not observed when IV bolus was given using a

central vein.

Thirty-one subjects were evaluable for efficacy: the subject with MDS had a hematological improvement (durable increase in hemoglobin as per International Working Group criteria for response in MDS) for 8 months. No objective tumor responses were observed, and 14 subjects had stable disease for at least 6 months.

## DISCUSSION

The objective of the "dose-determination" phase was to identify intravenous dose regimens of tipifarnib that would yield similar exposure relative to 200 mg administered orally, twice a day. More generally said, the objective was to identify an intravenous dose and schedule that would provide adequate exposure giving similar effects as the current oral administration. In the first and second cohorts with low-dose intravenous regimen, the systemic exposures to tipifarnib were considerably lower than those of 200 mg oral administration. Considered with the absence of dose limiting toxicity following these low-dose regimens, the final intravenous administration dose was escalated to 100 mg b.i.d. as a 2-hour infusion and 200 mg/day as a continuous infusion in the "bridging" phase of the study.

On average, the exposures ($AUC_{0-10h}$) to tipifarnib were comparable and within a narrow range (3990 to 4487 ng·h/mL) when tipifarnib was administered as an oral tablet (200 mg b.i.d.), 2-hour intravenous infusion (100 mg b.i.d.), or continuous intravenous infusion (200 mg/day). When the shape of the profiles of the 2 intravenous regimens is considered, the 2-hour infusion more closely mimicked oral administration.

The mean $C_{max}$ and $AUC_{0-10h}$ values following oral administration of 200 mg tipifarnib b.i.d. dose was 994 ng/mL and 3990 ng·h/mL, respectively. This is in good agreement with a previous study where 150- and 300-mg of tipifarnib was orally administered twice daily (mean $C_{max}$ of 443 and 974, respectively; mean $AUC_{0-12h}$ values of 2495 and 4674 ng·h/mL, respectively).

The mean oral bioavailability of tipifarnib tablet administered under fed conditions was 46.3% (by reference to 2-hour intravenous infusion b.i.d.) and 40.9% (by reference to continuous intravenous infusion). These results represent approximation of absolute oral bioavailability, since plasma samples were collected for only 10 hours after the last drug administration. However, it is clear that tipifarnib has relatively good bioavailability when administered with food.

Previous studies in humans indicted that tipifarnib undergoes extensive metabolism and unchanged parent compound is a relatively small constituent in plasma. Following oral administration, concentrations of unchanged tipifarnib were on average 42-times lower than total radioactivity concentrations in nonprecipitated samples and approximately 8-times lower in deproteinized samples. No unchanged drug was excreted in the urine and less than 7% of the oral dose administered was recovered in faces as tipifarnib. Consistent results were obtained in the present study. The plasma $AUC_{0-10h}$ values of tipifarnib-glucuronide were 3- to 30-fold higher than that of tipifarnib. Since tipifarnib possesses amino and keto groups in its structure, direct conjugation is to be expected. Importantly, in vitro studies with NIH 3T3 H-*ras* cell line and farnesyl protein transferase enzyme indicated that the glucuronide-metabolite of tipifarnib is not an inhibitor of farnesyltransferase. Other metabolites of tipifarnib formed via oxidative biotransfor<nation were measured in the plasma of patients including R130525 and R104209 and their respective glucuronidated conjugates, and R101763 in the present and previous studies. However, the concentrations of these metabolites were lower than those of tipifarnib.

Exposure to the two predominate metabolites of tipifarnib, the glucuronide conjugate and N-dealkylated species R130525, increased with an increase in the intravenous dose of tipifarnib administered. These pathways are apparently not saturated within the dose range studied. Relative exposure to R130525 after each route of administration was comparable. In contrast, the plasma concentrations of the glucuronidated metabolite were approximately 2-fold higher after oral dosing than after intravenous administration. More extensive glucuronidation during first-pass metabolism in the liver after oral administration is the most likely explanation of this route-dependent difference in the relative concentrations of tipifarnib-glucuronide. In addition, the metabolites identified in this study were independent of the administration routes. The results are also indicative a metabolite-specific impact of first-pass metabolism on the exposure to each metabolite.

The value of the plasma protein binding of tipifarnib from the *ex vivo* measurement on cancer subjects in this trial is very close to that (99.22% bound) obtained from an *in vitro* experiment with the plasma samples from 5 healthy men. In this *in-vitro* study, the blank plasma samples from these healthy subjects were fortified with $^{14}$C-tipifarnib at 1000 ng/mL and the measurement procedure was the same as one used in the current trial. The *in vitro* plasma protein binding of tipifarnib was also independent of the total tipifarnib concentrations in the range of 100 to 5000 ng/mL.

In conclusion, the plasma concentration-time profiles from the 2-hour intravenous regimen closely resembled the profiles after oral administration. Systemic exposure to tipifarnib was comparable for the first 10 hours after dosing when 100 mg was given twice daily as a 2-hour intravenous infusion or 200 mg was given via the oral route. No unusual adverse events were associated with intravenous tipifarnib.

## Claims

1. A pharmaceutical formulation suitable for intravenous administration comprising tipifarnib, mannitol, and hydroxypropyl-β-cyclodextrin.

2. The formulation of claim 1 in which the concentration of tipifarnib is 10 mg/ml.

## Patentansprüche

1. Zur intravenösen Verabreichung geeignete pharmazeutische Formulierung, die Tipifarnib, Mannitol und Hydroxypropyl-β-cyclodextrin umfasst.

2. Formulierung nach Anspruch 1, in der die Konzentration von Tipifarnib 10 mg/ml beträgt.

## Revendications

1. Formulation pharmaceutique qui convient pour l’administration intraveineuse comprenant tipifarnib, mannitol et hydroxypropyl-β-cyclodextrine.

2. Formulation selon la revendication 1, dans laquelle la concentration de tipifarnib est de 10 mg/ml.

**Table I.** Summary of Demographic Data

| Treatment Group | Dose-Determination Phase | | Bridging Phase | | Total |
|---|---|---|---|---|---|
| | Cohort 1 | Cohort 2 | Cohort 3 | | |
| | | | Sequence 1 | Sequence 2 | |
| Parameter | (n=3) | (n=3) | (n=12) | (n=14) | (N=32) |
| Sex, n (%) | | | | | |
| Male | 1 (33.3) | 1 (33.3) | 3 (25.0) | 5 (35.7) | 10 (31.3) |
| Female | 2 (66.7) | 2 (66.7) | 9 (75.0) | 9 (64.3) | 22 (68.8) |
| Age, yrs | | | | | |
| Median | 47 | 66 | 61 | 58.5 | 61 |
| Min-Max | 46-67 | 65-69 | 47-68 | 35-70 | 35-70 |
| Weight, kg | | | | | |
| Median | 59.5[1] | 68.5[1] | 58[3] | 62[3] | 61[6] |
| Min-Max | 52-67 | 57-80 | 48-92 | 48-88 | 48-92 |
| Height, cm | | | | | |
| Median | 171[2] | 160[2] | 165.5[4] | 164.5[5] | 164.5[7] |
| Min-Max | 171-171 | 160-160 | 149-174 | 150-176 | 149-176 |
| Race, n (%) | | | | | |
| White | 2 (66.7) | 3 (100) | 12 (100) | 13 (92.9) | 30 (93.8) |
| Black | 1 (33.3) | 0 | 0 | 1 (7.1) | 2 (6.3) |

[1] n=2. [2] n=1. [3] n=11. [4] n=10. [5] n=12. [6] n=26. [7] n=24.

**Table II.** Mean (±SD) Tipifarnib Plasma $C_{max}$ and $AUC_{0-10h}$ Values Following Administration as 2-Hour Intravenous Infusion b.i.d.

| Parameter | 30 mg n=3 | 60 mg n=3 | 100 mg n=24 |
|---|---|---|---|
| $C_{max}$, ng/mL[a] | 408 ± 81 | 702 ± 281 | 1382 ± 605 |
| $AUC_{0-10h}$, ng·h/mL | 1430 ± 308 | 2122 ± 1063 | 4487 ± 1344 |

[a] Plasma concentration of tipifarnib immediately before the end of the 2-h intravenous infusion.

**Table III.** Mean (±SD) Tipifarnib Plasma $C_{ss}$ and $AUC_{0\text{-}10h}$ Values Following Administration as Continuous Intravenous Infusion

| Parameter | 60 mg/day n=3 | 120 mg/day n=3 | 200 mg/day n=25 |
|---|---|---|---|
| Mean $C_{ss}$, ng /mL[a] | 154 – 175 | 204 – 230 | 333 – 641 |
| $AUC_{0\text{-}10h}$, ng·h/mL | 1640 ± 782 | 2155 ± 872 | 4449 ± 1931 |

[a] Mean plasma concentration of tipifarnib observed from 0 to 10 hours

**Table IV.** Systemic Exposure to Tipifarnib: 2-Hour Intravenous Infusion Versus Oral Dosing

| Parameter[c] | Oral b.i.d. 200 mg n=24 | 2-h i.v. b.i.d. 100 mg n=24 | Ratio (%)[b] | 90% Confidence interval |
|---|---|---|---|---|
| $t_{max}$, h | 2.0 | - | - | - |
| $C_{max}$, ng/mL | 994 ± 487 | 1382 ± 605 [d] | 148.0 | (122.1, 179.4) |
| $AUC_{0-10h}$, ng·h/mL | 3990 ± 1671 | 4487 ± 1344[e] | 119.3[f] | (100.5, 141.6)[f] |

[b] Ratio computed as the LSMEAN of the 2-hour intravenous to the LSMEAN of the oral administration multiplied by 100

[c] Mean ± SD values presented ($t_{max}$ values are medians)

[d] Plasma concentration of tipifarnib immediately before the end of the 2-h intravenous infusion

[e] n=22

[f] Only subjects (n=22) with complete PK sampling for 2-hour intravenous infusion b.i.d. and oral administration b.i.d. were included in this analysis.

**Table V**. Systemic Exposure to Tipifarnib: Continuous Intravenous Infusion Versus Oral Dosing

| Parameter[c] | Oral b.i.d. 200 mg n=24 | Continuous i.v. Infusion 200 mg/day n=25 | Ratio (%)[b] | 90% Confidence interval |
|---|---|---|---|---|
| $t_{max}$, h | 2.0 | - | - | - |
| $C_{max}$, ng/mL | 994 ± 487 | - | - | - |
| $AUC_{0-10h}$, ng·h/mL | 3990 ± 1671 | 4449 ± 1931 | 109.1[d] | (93.6, 127.1)[d] |

[b] Ratio computed as the LSMEAN of the continuous intravenous infusion to the LSMEAN of the oral administration multiplied by 100

[c] Mean ± SD values presented ($t_{max}$ values are medians)

[d] Only subjects (n=24) with complete PK sampling for continuous intravenous infusion and oral administration b.i.d. were included in this analysis

**Table VI.** Mean (±SD) Plasma Pharmacokinetic Parameters of Tipifarnib-Glucuronide Following Oral Administration, 2-Hour Intravenous Infusion, and Continuous Intravenous Infusion

| | Oral b.i.d. | 2-hour i.v. Infusion b.i.d. | | Continuous i.v. Infusion | |
|---|---|---|---|---|---|
| | 200 mg | 60 mg | 100 mg | 120 mg/day | 200 mg/day |
| Parameter | (n= 27) | (n=3) | (n=24) | (n=3) | (n=25) |
| $t_{max}$, h[a] | 3.3 | 2.3 | 3.1 | - | - |
| $C_{max}$, ng/mL | 9136±4644 | 2908±1794 | 4190±1552 | - | - |
| $AUC_{0-10h}$, ng·h/mL | 67716±40638 | 22172±15911 | 31731±13337 | 24612±21565 | 35507±18130 |
| $AUC_{0-10h}$ ratio[b] | 18.6±9.8 | 9.7±2.2 | 7.5±3.3 | 10.1±5.0 | 8.6±4.0 |

[a] $t_{max}$ values are medians.

[b] $AUC_{0-10h}$ ratio: metabolite/tipifarnib

**Table VII.** Mean (±SD) Plasma Pharmacokinetic Parameters of R130525 Following Oral Administration, 2-Hour Intravenous Infusion and Continuous Intravenous Infusion

| | Oral b.i.d. | 2-hour i.v. Infusion b.i.d. | | Continuous i.v. Infusion | |
|---|---|---|---|---|---|
| | 200 mg | 60 mg | 100 mg | 120 mg | 200 mg |
| Parameter | (n= 27) | (n=3) | (n=24) | (n=3) | (n=25) |
| $t_{max}$, h[a] | 2.0 | 2.3 | 2.3 | - | - |
| $C_{max}$, ng/mL | 133±66 | 29.2±14.2 | 62.8±26.1 | - | - |
| $AUC_{0-10h}$, ng·h/mL | 753±382 | 176±113 | 364±157 | 179±108 | 339±135 |
| $AUC_{0-10h}$ ratio[b] | 0.2±0.07 | 0.079±0.019 | 0.084±0.034 | 0.078±0.028 | 0.08±0.026 |

[a] $t_{max}$ values are medians.

[b] $AUC_{0-10h}$ ratio: metabolite/tipifarnib

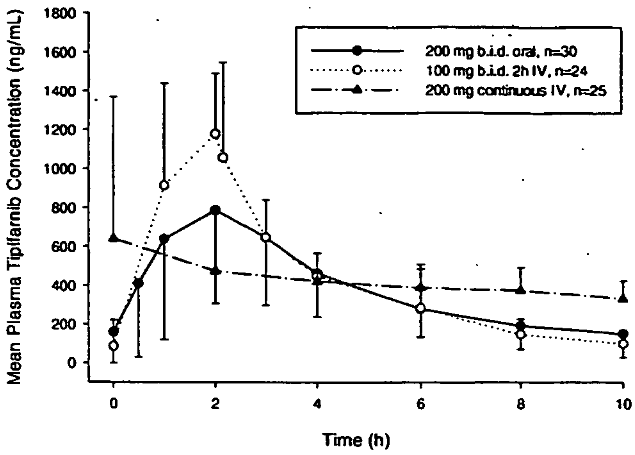
Mean Plasma Tipifarnib Concentration (ng/mL)
1800
1600
1400
1200
1000
800
600
400
200
0
0
2
4
6
8
10
Time (h)
200 mg b.i.d. oral, n=30
100 mg b.i.d. 2h IV, n=24
200 mg continuous IV, n=25

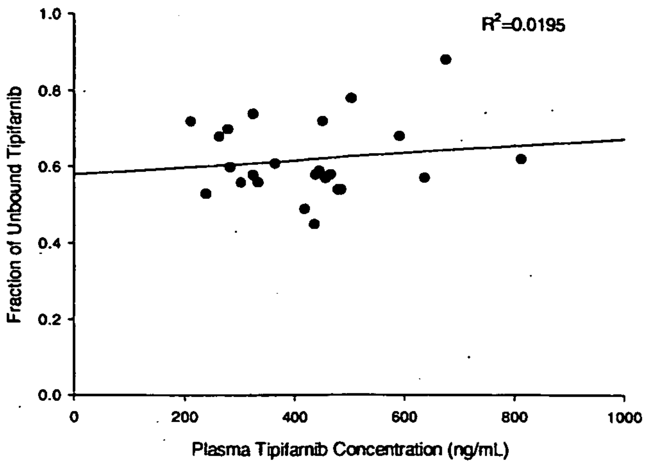
R²=0.0195
Fraction of Unbound Tipifarnib
1.0
0.8
0.6
0.4
0.2
0.0
0
200
400
600
800
1000
Plasma Tipifarnib Concentration (ng/mL)

Oral Administration (200 mg b.i.d.)

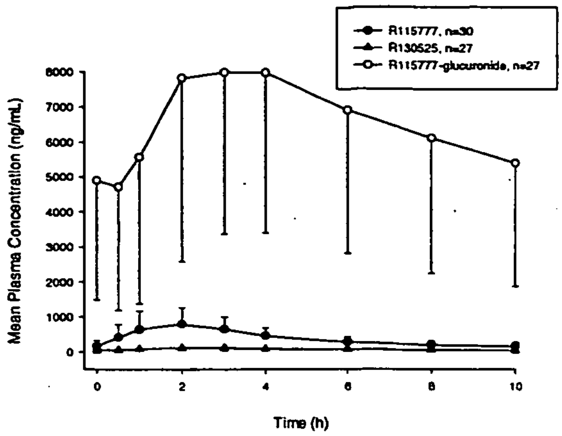


2-Hour Intravenous Infusion (100 mg b.i.d.)

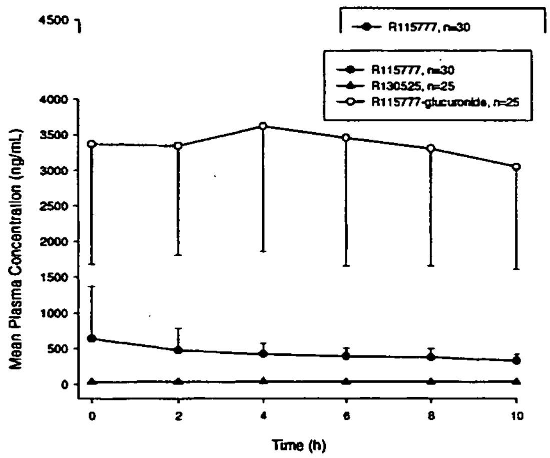


Continuous Intravenous Infusion (200 mg/day)

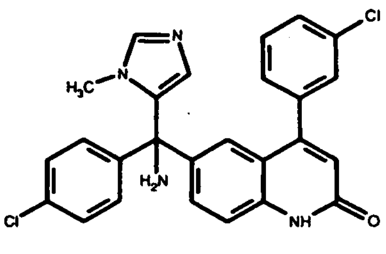

R130525

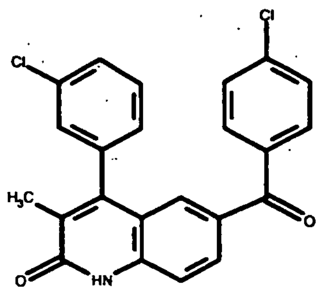

R107252

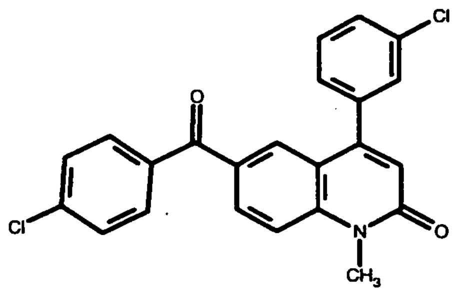

R104209

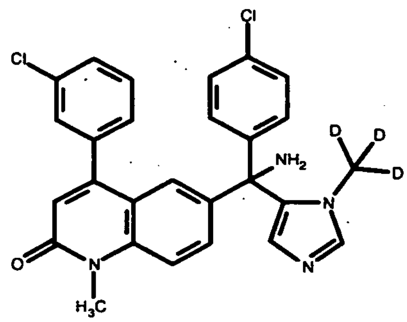

R198838

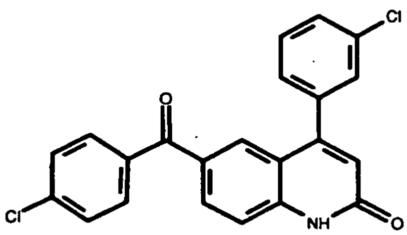

R101763

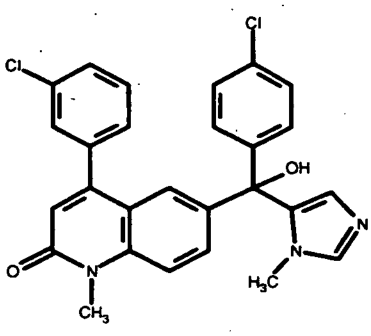

R121704

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9721701 A